# EUROPEAN PATENT SPECIFICATION

(11) **EP 0 818 952 B1**
(45) Date of publication and mention of the grant of the patent: **19.10.2005**
(21) Application number: 96909131.3
(22) Date of filing: 27.03.1996
(51) Int. Cl.: A01N 47/02, A01N 43/56

(54) **NEW METHOD OF COMBATING INSECTS**
NEUES VERFAHREN DER INSEKTENBEKÄMPFUNG
NOUVEAU PROCEDE DE LUTTE CONTRE LES INSECTES

(30) Priority: 05.04.1995 GB 9507073
(43) Date of publication of application: 21.01.1998
(73) Proprietor: BASF Agro B.V., Arnhem (NL)-Wädenswil-Branch, 8820 Wädenswil/Au (CH)
(72) Inventor: TWINN, David, C. Research Station of, Ongar, Essex CM5 0HW (GB)
(74) Representative: Pohl, Michael Friedrich
(86) International application number: PCT/EP1996/001335
(87) International publication number: WO 1996/031123

(56) References cited:
- EP-A- 0 201 852
- EP-A- 0 295 117
- EP-A- 0 303 118
- EP-A- 0 398 499
- EP-A- 0 500 209
- EP-A- 0 679 650
- WO-A-92/13451

## Description

This invention relates to a new method of combating insects such as cockroaches or ants at a locus connected to public health, that is to say at a locus frequented by humans, either private or public.

Many insecticidally active compounds are known, such as the insecticidal pyrazoles described in International Patent Publications No. WO 87/03781, WO 93/06089 and WO 94/21606, as well as in European Patent Publications 0295117, 0403300, 0385809, and 0679650, German Patent Publication 19511269 and United States Patents 5,232,940 and 5,236,938. The use of such compounds to kill cockroaches or ants has already been contemplated, but such use was essentially in connection with baits or ingested materials or food that the insect is supposed to eat, or also in connection with direct contact application to the said insects.

The use of baits can be problematic because there is a need to place the baits at a proper locus where the cockroaches are supposed to come. Furthermore the baits can become a safety hazard to children, an undesirable situation.

An object of the instant invention is to provide a method of control of cockroaches or ants avoiding the use of baits or ingestible material.

An object of the instant invention is to provide a method of control of cockroaches or ants avoiding the direct application of the active material to the insect itself, as with baits for example.

The object of the instant invention is to provide a simplified and efficient method of control of cockroaches or ants.

The present invention is thus directed to the use of 1-phenylpyrazole derivatives selected from
5-amino-3-cyano-1-(2,6-dichloro-4-trifluoromethylphenyl)-4-trifluoromethylsulfinyl pyrazole (hereafter referred to as compound A).
5-amino-3-cyano-1-(2,6-dichloro-4-trifluoromethylphenyl)-4-trifluoromethylthio pyrazole (hereafter referred to as compound B) and
5-amino-3-cyano-1-(2,6-dichloro-4-trifluoromethylphenyl)-4-trifluoromethylsulfonyl pyrazole (hereafter referred to as compound C),
in the form of a thin dry layer or embedded in a thin dry layer, which is applied to a surface on which the insect is moving or expected to move, which layer the insect is not able to seize or bite or eat directly,
the surface being selected from glass, ceramics, concrete, plastics surface, metallic or wooden surface, and textiles,
the thin dry layer containing an effective amount of the 1-phenylpyrazole derivative, the effective amount being from 0.01 g to 10 g per 100 square meters
for obtaining lasting control of cockroaches or ants or of a population of cockroaches or ants.

It should be understood that in the instant invention, the active ingredient is rather in the form of a thin layer or imbedded in a thin layer, and that this layer is covering totally or partially the surface where the insect is making contact or is moving or walking or going to walk or expected to be walking or supposed to be walking. Due to this thin layer the insect is not able to seize or bite or eat directly a discrete volume of composition comprising the ingredient of formula (I). This is connected with the unobviousness of the invention.

According to a further aspect of the invention, it provides a method of control of a population of cockroaches or ants able to walk or travel in public or private housing or building or household or home, whereby a non seizable, but insecticidally effective, amount of active ingredient is lying on a surface located in the area to be treated.

According to a further aspect of the invention, it provides a method of control of a population of cockroaches or ants able to walk or travel in public or private housing or building or household or home, whereby a thin layer is covering the surface where the cockroaches or ants are supposed to walk.

According to a further aspect of the invention, it provides a method of control of a population of cockroaches or ants that are able to walk or travel in public or private housing or building or household or home, whereby the said cockroaches or ants are caused to walk on a thin layer covering a surface in or near the area where the said insects are to be killed.

According to a preferred and most efficient embodiment, the invention is applied specifically to cockroaches.

The method of the invention is especially advantageous because it is very easy to apply the active ingredient, preferably by mean of spraying a liquid formulation to the appropriate surface.

The surface which is treated according to the invention may be smooth or rough or rugged. Smooth surfaces are more effective. The surface to be treated is selected from glass, ceramics, concrete ; plastics surface such as vinyl plastics, melamine, linoleum ; metallic or wooden surface such as furniture : textiles such as clothes.

The deposited layer of active ingredient according to the invention may be wet just after application, or may be dried or dry sometime after. The creation of this layer may be made by all known methods of covering, for example as a spray coat, paint, dip, wash, soak, lacquer, foam, dust, powder, aqueous suspension, paste, cream, wettable powder, aerosol, emulsifiable concentrate, concentrated suspension, flowable suspension, aqueous suspension, oil suspension, oil solution, pressure pack, or other standard formulation well known by those skilled in the art.

Compositions comprising an active ingredient of formula (I), especially liquid compositions, have already been described in the here above cited prior art.

Treatment of cockroaches in public health in public housing or building for the control of so-called American cockroaches (Periplaneta americana), but also of other cockroaches like German cockroaches (Platella germanica), is a preferred feature of the instant invention.

The invention accordingly provides a method for controlling cockroaches or ants which comprises applying to a surface with which the cockroaches or ants make contact or are to make contact a compound of formula L The compound is preferably applied as a thin layer.

The preparation of compounds of formula (I) may be performed according to any process described in the here above cited patent applications, or other process according to the knowledge of a person skilled in the art of chemical synthesis.

The effective compositions which may be used in the invention may be offered or presented in different amounts comprising the 1-phenylpyrazole derivative in an amount between 0.01 g and 10 g/100 m².

The compositions which are useful in the invention (and which are to be spread on surfaces to control cockroaches or ants) comprise generally 0.0001 up to 15 % w/w of active ingredient, preferably from 0.1 to 6 w/w. They may be in the form of a liquid before application, especially in the form of an emulsifiable concentrate, aqueous emulsion, concentrated suspension or flowable suspension. But after drying of the liquid in the form of a thin layer.

The insecticidal compositions may also contain all kind of compatible surface active agent and/or carrier. Adjuvants may also be used, such as sticking agents, dyestuffs, film forming agent or the like. The carrier itself may be solid or liquid.

The compounds of formula (I) may be used in sequence or admixture, particularly admixtures with another pesticide e.g. an insecticide, acaricide or fungicide.

The compositions may be prepared by admixing the ingredients.

The invention is illustrated by the following examples which should not be considered as limiting or restricting the invention.

### EXAMPLE 1

Compounds A and B were dissolved in an acetone/water mixture and deposited on glass and left overnight to dry. The following results were observed :

| COMPOUND | DOSE LEVEL mg/100 m² | Mortality % 90 min. after introduction of the cockroaches | Mortality % 18 h. after introduction of the cockroaches |
|---|---|---|---|
| B | 125 | 100 | 100 |
| B | 31 | 100 | 100 |
| B | 10 | 0 | 91 |
| A | 125 | 100 | 100 |
| A | 31 | 100 | 100 |
| A | 10 | 5 | 100 |

### Example 2

Compound A was formulated as a 12.5 grams per liter liquid formulation in three compositions: (i) an emulsifiable concentrate (EC-wetting agent and an organic solvent); (ii) an aqueous emulsion (EW- wetting agent plus water plus an organic solvent); and (iii) a concentrated suspension (SC- dispersing agent and water) . Each formulation was dissolved in water and deposited on samples of painted cement and plastic flooring (as described in Méthode C.E.B. n° 159 Chapitre I I / § 1, "Méthode d'essai d'efficacité practique de spécialités insecticides destinées a la destruction des blattes dans les locaux Novembre 1992," paragraph 2.6.) to provide deposits of 125 mg/100 m², 500 mg/100 m², and 1250 mg/100 m². *Blattella germanica* (German cockroaches) between 2 and 15 days old were placed on the surfaces for four hours and then removed, put in an untreated jar and observed after 96 hours from the beginning of the exposure. Each treatment was replicated 3 times. The following results were observed. Mortalities are reported as percentages of dead insects among living ones.

| Formulation / Dose | Mortality % on Plastic | Mortality % on Cement |
|---|---|---|
| EC /125 mg/100 m² | 10.2 | 100 |
| EC /500 mg/100 m² | 100 | 100 |
| EC /1250 mg/100 m² | 100 | 100 |
| EW /125 mg/100 m² | 93.3 | 100 |
| EW /500 mg/100 m² | 88 | 95.7 |
| EW /1250 mg/100 m² | 100 | 100 |
| SC /125 mp/100 m² | 93.3 | 100 |
| SC /500 mg/100 m² | 67.7 | 100 |
| SC / 1250 mg/100 m² | 100 | 100 |

### Example 3

The samples of plastic flooring and cement of Example 2 were held at 25°C in the dark for 24 hours. The infestation and observations of Example 2 were repeated. The following results were observed:

| Formulation / Dose | Mortality % on Plastic | Mortality % on Cement |
|---|---|---|
| EC /125 mg/100 m² | 14 | 100 |
| EC /500 mg/100 m² | 100 | 100 |
| EC /1250 mg/100 m² | 100 | 100 |
| EW /125 mg/100 m² | 49 | 100 |
| EW /500 mg/100 m² | 74 | 100 |
| EW /1250 mg/100 m² | 100 | 100 |
| SC /125 mg/100 m² | 43 | 100 |
| SC /500 mg/100 m² | 100 | 100 |
| SC /1250 mg/100 m² | 100 | 100 |

### Example 4

The samples of plastic flooring and cement of Example 2 were held at 25°C in the dark for 14 days. The infestation and observations of Example 2 were repeated. The following results were observed:

| Formulation / Dose | Mortality % on Plastic | Mortality % on Cement |
|---|---|---|
| EC /125 mg/100 m² | 13.6 | 100 |
| EC /500 mg/100 m² | 100 | 100 |
| EC /1250 mg/100 m² | 100 | 100 |
| EW /125 mg/100 m² | 37.7 | 100 |
| EW /500 mg/100 m² | 77.7 | 100 |
| EW /1250 mg/100 m² | 100 | 100 |
| SC /125 mg/100 m² | 44.3 | 100 |
| SC /500 mg/100 m² | 100 | 100 |
| SC /1250 mg/100 m² | 100 | 100 |

### Example 5

The samples of plastic flooring and cement of Example 2 were held at 25°C in the dark for 30 days. The infestation and observations of Example 2 were repeated. The following results were observed:

| Formulation / Dose | Mortality % on Plastic | Mortality % on Cement |
|---|---|---|
| EC /125 mg/100 m² | 6.7 | 72.3 |
| EC /500 mg/100 m² | 100 | 100 |
| EC /1250 mg/100 m² | 100 | 100 |
| EW /125 mg/100 m² | 53.3 | 100 |
| EW /500 mg/100 m² | 82.3 | 100 |
| EW / 1250 mg/100 m² | 100 | 100 |
| SC /125 mg/100 m² | 51 | 100 |
| SC /500 mg/100 m² | 100 | 100 |
| SC /1250 mg/100 m² | 100 | 100 |

### Example 6

The samples of plastic flooring and cement of Example 2 were held at 25°C in the dark for 90 days. The infestation and observations of Example 2 were repeated. The following results were observed:

| Formulation / Dose | Mortality % on Plastic | Mortality % on Cement |
|---|---|---|
| EC /125 mg/100 m² | 8.9 | 24.3 |
| EC /500 mg/100 m² | 95.7 | 100 |
| EC /1250 mg/100 m² | 100 | 100 |
| EW /125 mg/100 m² | 69 | 100 |
| EW /500 mg/100 m² | 95.7 | 100 |
| EW / 1250 mg/100 m² | 100 | 100 |
| SC /125 mg/100 m² | 84.3 | 100 |
| SC / 500 mg/100 m² | 100 | 100 |
| SC /1250 mg/100 m² | 100 | 100 |

### Example 7

The samples of plastic flooring and cement of Example 2 were held at 25°C in the dark for 120 days. The infestation and observations of Example 2 were repeated. The following results were observed:

| Formulation / Dose | Mortality % on Plastic | Mortality % on Cement |
|---|---|---|
| EC /125 mg/100 m² | 4.3 | 8.8 |
| EC /500 mg/100 m² | 95.7 | 97.7 |
| EC /1250 mg/100 m² | 100 | 100 |
| EW /125 mg/100 m² | 69 | 100 |
| EW /500 mg/100 m² | 88.7 | 100 |
| EW / 1250 mg/100 m² | 100 | 100 |
| SC /125 mg/100 m² | 86.7 | 100 |
| SC / 500 mg/100 m² | 100 | 100 |
| SC /1250 mg/100 m² | 100 | 100 |

### Example 8

The samples of plastic flooring and cement of Example 2 were held at 25°C in the dark for 180 days. The infestation and observations of Example 2 were repeated. The following results were observed:

| Formulation / Dose | Mortality % on Plastic | Mortality % on Cement |
|---|---|---|
| EC /125 mg/100 m² | 0 | 0 |
| EC /500 mg/100 m² | 100 | 100 |
| EC /1250 mp/100 m² | 100 | 100 |
| EW /125 mg/100 m² | 60 | 100 |
| EW /500 mg/100 m² | 93.3 | 100 |
| EW /1250 mg/100 m² | 100 | 100 |
| SC /125 mg/100 m² | 24.4 | 75.5 |
| SC /500 mg/100 m² | 100 | 100 |
| SC /1250 mg/100 m² | 100 | 100 |

### Example 9

Porous concrete is impregnated with a 20 ppm solution acetone/water solution of Compound B and cockroaches (*Blattella germanica*) were permitted to walk on the surface. One day after treatment, the insects were observed. 50% of the insects had died.

## Claims

1. Use of 1-phenylpyrazole derivatives selected from
5-amino-3-cyano-1-(2,6-dichloro-4-trifluoromethylphenyl)-4-trifluoromethylthio pyrazole,
5-amino-3-cyano-1-(2,6-dichloro-4-trifluoromethylphenyl)-4-trifluoromethylsulfinyl pyrazole and
5-amino-3-cyano-1-(2,6-dichloro-4-trifluoromethylphenyl)-4-trifluoromethylsulfonyl pyrazole,
in the form of a thin dry layer or embedded in a thin dry layer, which is applied to a surface on which the insect is moving or expected to move, which layer the insect is not able to seize or bite or eat directly,
the surface being selected from glass, ceramics, concrete, plastics surface, metallic or wooden surface, and textiles,
the thin dry layer containing an effective amount of the 1-phenylpyrazole derivative, the effective amount being from 0.01 g to 10 g per 100 square meters
for obtaining lasting control of cockroaches or ants or of a population of cockroaches or ants.

2. The use according to claim 1 comprising control of a population of cockroaches or ants able to walk or travel in public or private housing or buildings or household or home.

3. The use according to claim 1 or 2 comprising control of a population of cockroaches or ants able to walk or travel in public or private housing or buildings or household or home, wherein the said cockroaches or ants are caused to walk on a thin layer covering a surface in or near the area where the said insects are to be killed.

4. Use according to any one of the foregoing claims when applied to cockroaches.

5. Use according to any one of the foregoing claims wherein the 1-phenylpyrazole derivative is applied as an insecticidal composition, which comprises from 0.0001 to 15% w/w of 1-phenylpyrazole derivative.

6. Use according to claim 5, wherein the insecticidal composition comprise from 0.1 to 6% w/w of 1-phenylpyrazole derivative.

## Patentansprüche

1. Verwendung von 1-Phenylpyrazole-Derivaten, ausgewählt unter
5-Amino-3-cyano-1-(2,6-dichlor-4-trifluormethylphenyl)-trifluormethylthiopyrazol
5-Amino-3-cyano-1-(2,6-dichlor-4-trifluormethylphenyl)-trifluormethylsulfinylpyrazol und
5-Amino-3-cyano-1-(2,6-dichlor-4-trifluormethylphenyl)-trifluormethylsulfonylpyrazol,
in Form einer dünnen, trockenen Schicht oder eingebettet in eine dünne trockene Schicht, die auf einer Oberfläche aufgebracht wird, auf welcher sich das Insekt bewegt oder erwartungsgemäß bewegen wird, wobei das Insekt nicht in der Lage ist, die Schicht zu ergreifen oder beißen oder direkt zu essen,
wobei die Oberfläche unter Glas, Keramik, Zement, Plastikoberflächen, Metall- oder Holzoberflächen und Textil ausgewählt ist,
wobei die dünne trockene Schicht eine wirksame Menge des 1-Phenylpyrazol Derivats enthält, wobei die wirksame Menge 0,01 bis 10 g pro 100 Quadratmeter beträgt, zu Erzielung einer dauerhaften Kontrolle von Schaben oder Ameisen oder Popolation von Schaben oder Ameisen.

2. Verwendung nach Anspruch 1, umfassed die Kontrolle einer Population von Schaben oder Ameisen, die in der Lage sind, sich in öffentlichen oder privaten Wohngebäuden, Gebäuden, im Haushalt oder in Wohnungen zu bewegen.

3. Verwendung nach Anspruch 1 oder 2, umfassed die Kontrolle einer Population von Schaben, die in der Lage sind, sich in öffentlichen oder privaten Wohngebäuden, Gebäuden, im Haushalt oder in Wohnungen zu bewegen, wobei die Schaben oder Ameisen veranlasst werden, sich auf einer dünnen Schicht zu bewegen, die eine Oberfläche in oder nahe dem Bereich, in welchem die Insekten getötet werden sollen, bedeckt.

4. Verwendung nach einem der vorhergehenden Ansprüche zur Anwendung gegen Schaben.

5. Verwendung nach einem der vorhergehenden Ansprüche, wobei das 1-Phenylpyrazol-Derivat als Insektizid-Zusammensetzung, die 0,0001 bis 15 Gew.% des 1-Phenylpyrazol-Derivats enthält, angewendet wird.

6. Verwendung nach Anspruch 5, wobei die Insektizid-Zusammensetzung 0,1 bis 6 Gew.-% des 1-Phenylpyrazol-Derivats enthält.

## Revendications

1. Utilisation de dérivés de 1-phénylpyrazole choisis parmi
du 5-amino-3-cyano-1-(2,6-dichloro-4-trifluorométhylphényl)-4-trifluorométhylthiopyrazole,
du 5-amino-3-cyano-1-(2,6-dichloro-4-trifluorométhylphényl)-4-trifluorométhylsulfinylpyrazole, et
du 5-amino-3-cyano-1-(2,6-dichloro-4-trifluorométhylphényl)-4-trifluorométhylsulfonylpyrazole,
sous la forme d'une couche sèche, mince, ou noyés dans une couche sèche mince, qui est appliquée sur une surface sur laquelle l'insecte se déplace ou il est attendu qu'il se déplace, l'insecte n'étant pas capable de s'emparer ou de piquer ou de manger directement cette couche,
la surface étant choisie parmi des surfaces de verre, de matières céramiques, de ciment, de matières plastiques, de métal ou de bois et des matières textiles,
la couche sèche mince contenant une quantité efficace du dérivé de 1-phénylpyrazole, la quantité efficace étant de 0,01 g à 10 g par 100 mètres carrés pour obtenir un contrôle durable des blattes ou des fourmis ou d'une population de blattes ou de fourmis.

2. Utilisation suivant la revendication 1, comprenant un contrôle d'une population de blattes ou de fourmis capables de se promener ou de se déplacer dans des logements publics ou privés ou des constructions ou des ménages ou des maisons.

3. Utilisation suivant la revendication 1 ou 2, comprenant un contrôle d'une population de blattes ou de fourmis capables de se promener ou de se déplacer dans des logements publics ou privés ou des constructions ou des ménages ou des maisons, dans laquelle lesdites blattes ou fourmis sont obligées de se promener sur une couche mince couvrant une surface dans ou à proximité de la zone où lesdits insectes doivent être tués.

4. Utilisation suivant l'une quelconque des revendications précédentes, lorsqu'elle est appliquée aux blattes.

5. Utilisation suivant l'une quelconque des revendications précédentes, dans laquelle le dérivé de 1-phénylpyrazole est appliqué comme composition insecticide qui comprend de 0,0001 à 15 % en poids/poids de dérivé de 1-phénylpyrazole.

6. Utilisation suivant la revendication 5, dans laquelle la composition insecticide comprend de 0,1 à 6 % en poids/poids de dérivé de 1-phénylpyrazole.
